# EUROPÄISCHE PATENTSCHRIFT

(11) **EP 3 717 878 B1**
(45) Veröffentlichungstag und Bekanntmachung des Hinweises auf die Patenterteilung: **17.01.2024**
(21) Anmeldenummer: 18795422.7
(22) Anmeldetag: 23.10.2018
(51) Int. Cl.: G01K 1/08, G01K 1/14, G01K 13/02, G01D 11/24, G01D 11/26, G01N 25/66, G01N 33/00

(54) **SENSORMODUL ZUR LUFTGÜTEMESSUNG**
SENSOR MODULE FOR AIR QUALITY MEASUREMENT
MODULE DE CAPTEURS POUR LA MESURE DE LA QUALITÉ DE L'AIR

(30) Priorität: 15.01.2018 DE 202018100186 U
(43) Veröffentlichungstag der Anmeldung: 07.10.2020
(73) Patentinhaber: Sensirion AG, 8712 Stäfa (CH)
(72) Erfinder: BRAUN, Stephan, 8712 Stäfa (CH); SCHMID, Tobias, 8712 Stäfa (CH); BRUGGER, Thomas, 8712 Stäfa (CH); MERZ, Matthias, 8712 Stäfa (CH); BECKER, Manuel, 8712 Stäfa (CH)
(74) Vertreter: Toleti, Martin
(86) Internationale Anmeldenummer: PCT/EP2018/078997
(87) Internationale Veröffentlichungsnummer: WO 2019/137647

(56) Entgegenhaltungen:
- DE-B3-102005 016 896
- DE-C1- 19 734 110
- DE-T5-112013 001 060
- DE-T5-112014 003 199
- DE-U- 1 827 060
- JP-A- 2016 196 857

## Beschreibung

### Gebiet der Erfindung

Die Erfindung betrifft ein Sensormodul sowie ein Verfahren zur Herstellung eines Sensormoduls zur Bestimmung einer Eigenschaft eines Fluids, insbesondere zur Luftgütemessung, im Besonderen in einem Innenraum oder einem Luftkanal eines Kraftfahrzeugs oder eines Gebäudes.

### Hintergrund

Sensormodule zur Luftgütemessung messen verschiedene Parameter, die als charakteristisch für die Güte der umgebenden Luft angesehen werden. Dazu gehören beispielsweise Temperatur, relative Feuchtigkeit, Aerosolkonzentration oder eine Konzentration von verschiedenen Gasen wie Ozon, Stickstoffdioxid, Schwefeldioxid oder Kohlenmonoxid. Sensormodule zur Luftgütemessung werden zur Messung und Überwachung von Luftparametern eingesetzt, z.B. in städtischen Ballungsräumen. Außerdem werden sie bei der Messung von Luftparametern in Innenräumen verwendet, z.B. in Gebäuden oder Kraftfahrzeugen. Ein spezieller Anwendungsbereich ist die Luftzufuhr für solche Innenräume, wobei die Messwerte der Luftparameter zur Steuerung und Herbeiführung eines angenehmen Klimas im Innenraum herangezogen werden. Sensormodule zur Luftgütemessung werden aber auch in Außenluftmessungen eingesetzt, z.B. an Gebäuden oder Kraftfahrzeugen.

Herkömmliche Sensoranordnungen haben teilweise große Abmessungen, umfassen mehrere, nicht integrierte Einzelsensoren, sind empfindlich gegen mechanische Beschädigung oder aufwändig zu installieren, benötigen mehrere Kammern für die Messung verschiedener Luftparameter oder sind ungenau. DE 11 2013 001 060 T5 offenbart eine herkömmliche Luftmengenmesseinrichtung. Die Messeinrichtung der DE'060 weist einen Leiterrahmen mit einem Strömungsratensensorchip auf. Dieser Leiterrahmen ist mit einem Kapselungsaufbau ausgestattet, der mit Ausnahme eines Teils der Oberfläche des Strömungsratensensorchips mit einem Harz bedeckt ist.

### Darstellung der Erfindung

Der vorliegenden Erfindung liegt die Aufgabe zugrunde, die Nachteile der herkömmlichen Sensoranordnungen zu beheben bzw. eine vorteilhafte alternative Ausführungsform bereitzustellen. Diese Aufgabe wird durch ein Sensormodul gemäß dem Anspruch 1 und durch ein Verfahren zur Herstellung eines Sensormoduls gemäß dem Anspruch 11 gelöst.

Das Sensormodul umfasst eine Leiterplatte, mindestens einen Sensor auf der Leiterplatte zum Aufnehmen einer Messgröße eines Fluids, insbesondere der umgebenden Luft, und ein Gehäuse für die Leiterplatte. Das Fluid kann dabei in gasförmigem Zustand, oder flüssigem Zustand, oder als Aerosol vorliegen.

Dabei ragt ein Teil der Leiterplatte aus einer Öffnung im Gehäuse heraus, wobei sich der mindestens eine Sensor auf einer Vorderseite des herausragenden Teils der Leiterplatte befindet. Zumindest die Vorderseite des herausragenden Teils der Leiterplatte mit Ausnahme einer Aussparung für den mindestens einen Sensor ist mit einer Füllmasse vergossen. Über die Aussparung kann der mindestens eine Sensor direkt in Kontakt mit dem Fluid, beispielsweise mit der umgebenden Luft stehen.

Zusätzlich zur Vorderseite kann auch eine der Vorderseite gegenüberliegende Rückseite des herausragenden Teils der Leiterplatte mit Ausnahme einer weiteren Aussparung, die dem mindestens einen Sensor gegenüberliegt, mit der Füllmasse vergossen sein. Alternativ kann auch ein Teil des Gehäuses die Rückseite der Leiterplatte zumindest teilweise unterstützen und mit der Leiterplatte vergossen sein. Außerdem ist zusätzlich zumindest die Öffnung im Gehäuse, aus der die Leiterplatte herausragt, mit der Füllmasse vergossen.

In einer Ausführungsform befindet sich auch auf der Rückseite der Leiterplatte mindestens ein Sensor.

Das Gehäuse ist bevorzugt aus einem festen Material gefertigt, im Besonderen aus Kunststoff, z.B. aus PBT oder PP, wodurch das Sensormodul robust ist. Das Gehäuse und die Leiterplatte mit dem mindestens einen Sensor sind bevorzugt einzeln gefertigt und dann zusammengesetzt. Wenn die Leiterplatte in das Gehäuse eingesetzt ist, ragt der Teil mit dem Sensor zur Messung der Luftgüte an einer Seite, die als Vorderseite des Gehäuses bezeichnet wird, aus dem Gehäuse heraus. Das Vergießen der Leiterplatte mit Füllmasse verstärkt die Leiterplatte und dichtet sie ab, sodass ein robustes Sensormodul entsteht.

Darüber hinaus macht das Vergießen der Leiterplatte eine Messkammer für den mindestens einen Sensor überflüssig, die aufgrund des eingeschlossenen Volumens die Messung einerseits träge machen würde und andererseits zu falschen Messungen infolge von angesammelten Ausgasungen aus dem Sensormodul selbst führen könnte. Somit ermöglicht das vorliegende Sensormodul eine schnelle und genaue Messung der zu bestimmenden Grösse, beispielsweise der Luftgüte.

In einer bevorzugten Ausführungsform ist ein Grundkörper des Gehäuses quaderförmig. Der quaderförmige Teil dient als Aufnahme für die Leiterplatte. Je nach Verwendungszweck und Anschlussmöglichkeiten kann das Gehäuse weitere Teile umfassen. Beispielteile werden im Folgenden beschrieben.

Das Gehäuse umfasst bevorzugt einen Stecker, über den die Leiterplatte von außerhalb des Sensormoduls elektrisch kontaktiert ist. In einer bevorzugten Ausführungsform ist der Stecker ein Standardstecker mit elektrischen Kontakten, über die eine Stromversorgung und ein Auslesen der Messdaten des mindestens einen Sensors bewerkstelligt werden. Der Stecker kann sich beispielsweise an einer Seite des Gehäuses befinden, die an die Vorderseite mit der Öffnung angrenzt oder ihr gegenüberliegt.

Weiterhin kann das Gehäuse zur Anbringung, z.B. an einem Luftkanal oder in einem Wasserkasten, ein Befestigungsmittel umfassen. Der Luftkanal kann beispielsweise die Luftzufuhr für einen Innenraum oder ein Rohr in einer Messanlage sein, das eine Öffnung zur Aufnahme des Sensormoduls aufweist. Das Befestigungsmittel befindet sich bevorzugt an der Vorderseite des Gehäuses. Bevorzugt hat das Befestigungsmittel die Form einer Steckverbindung, z.B. eines Bajonettverschlusses. Das Befestigungsmittel ist bevorzugt so ausgeformt, dass in Betriebsposition der Teil der Leiterplatte mit dem mindestens einen Sensor in den Luftkanal hineinragt, während sich der Stecker außerhalb des Luftkanals befindet.

Das Gehäuse kann eine weitere Öffnung auf einer Seite umfassen, die an die Vorderseite mit der ersten Öffnung angrenzt. Die weitere Öffnung kann beispielsweise benutzt werden, um die Leiterplatte mit dem mindestens einen Sensor einfach in das Gehäuse einzuführen. Im fertigen Zustand ist die weitere Öffnung bevorzugt ebenfalls mit der Füllmasse vergossen.

Weiterhin kann die gesamte Leiterplatte mit der Füllmasse vergossen sein, was ein kompaktes und abgedichtetes Sensormodul ermöglicht, und welches Vergiessen bevorzugt in einem Arbeitsschritt erfolgt, sodass die Vergussmasse einteilig ausgebildet ist. Ausgenommen davon sind lediglich eine Aussparung für den mindestens einen Sensor und eine weitere Aussparung, die dem mindestens einen Sensor gegenüberliegt und herstellungsbedingt entsteht, wie weiter unten erläutert wird. In einer anderen Ausführungsform kann ein Teil der oder die gesamte Rückseite der Leiterplatte vom Gehäuse abgedeckt sein.

Bevorzugt weist das Gehäuse zum Einführen der Leiterplatte durch die weitere Öffnung eine Führungsvorrichtung auf. Im Besonderen kann die Führungsvorrichtung eine Abschrägung zum schrägen Einführen der Leiterplatte in das Gehäuse umfassen, wobei die Abschrägung schräg zur Seite mit der weiteren Öffnung angeordnet ist. Beim Einführen der Leiterplatte wird darauf geachtet, dass erhabene Bauteile wie Sensorchips auf der Leiterplatte nicht beschädigt werden. Ein sachgemäßes Einführen erfolgt mit genügend Abstand zur Abschrägung. Im nächsten Schritt wird die Leiterplatte bevorzugt auf die Abschrägung gedrückt. Abschließend wird die Leiterplatte über die Abschrägung in ihre finale Position im Gehäuse gekippt.

Außerdem kann das Gehäuse zum Arretieren der Leiterplatte nach dem Einführen eine Arretierungsvorrichtung umfassen. Die Arretierungsvorrichtung kann als Kunststoffklammer im Gehäuse ausgebildet sein und hält die Leiterplatte für den Schritt des Vergießens in ihrer finalen Position. Denkbar sind zur Arretierung auch das Verschweißen oder Verpressen eines mit dem Gehäuse verbundenen Pins auf der Leiterplatte oder das Verlöten oder Verpressen eines fest mit dem Gehäuse verbundenen Steckerpins auf der Leiterplatte.

Bevorzugt misst ein Sensor des Sensormoduls eine Temperatur eines Fluids, beispielsweise der umgebenden Luft. Der Temperatursensor kann beispielsweise einen temperaturabhängigen elektrischen Widerstand enthalten. In einer anderen Ausführungsform misst ein Sensor eine relative Feuchtigkeit eines Fluids, beispielsweise der umgebenden Luft. Im Besonderen können der Temperatursensor und der Feuchtesensor in einem einzigen Chip integriert sein, wodurch beispielsweise ein Taupunkt der umgebenden Luft bestimmt werden kann.

Temperatur und relative Feuchtigkeit der Luft sind wichtige Parameter für die Luftgüte und ein angenehmes Klima. Daher wird das Sensormodul bevorzugt zur Messung der Luftgüte in einem Luftkanal oder einem Innenraum, im Besonderen eines Kraftfahrzeugs oder eines Gebäudes, eingesetzt. Darüber hinaus kann ein Messwert zur Steuerung des Klimas in dem Innenraum verwendet werden, was beispielsweise über die Steuerung der Luftzufuhr oder eine Klimaanlage bewerkstelligt wird.

In einer anderen Ausführungsform ist ein Sensor des Sensormoduls ein Gassensor, der bevorzugt eine Konzentration eines Gases in einem umgebenden Fluid misst, beispielsweise in der umgebenden Luft. Der oben genannte Temperatursensor oder die Kombination aus Temperatur- und Feuchtesensor kann auch durch einen solchen Gassensor ergänzt werden. Die Konzentration von verschiedenen Gasen kann ebenfalls eine wichtige Eigenschaft oder Parameter eines Fluids sein, den es zu bestimmen gilt, beispielsweise die Luftgüte. So ist beispielsweise eine erhöhte Konzentration von Ozon, Stickstoffdioxid, Schwefeldioxid oder Kohlenmonoxid gesundheitsschädlich. Messwerte des Gassensors können daher ebenfalls zur Steuerung der Luftzufuhr oder der Klimaanlage verwendet werden. So kann beispielsweise die Luftzufuhr von außen abgestellt werden, wenn im Luftzufuhrkanal eine erhöhte Konzentration an gesundheitsschädlichen Gasen festgestellt wird.

Bevorzugt ist der Sensor zur Messung einer Gaskonzentration ein MOX-Sensor (Metalloxid). Zur Erreichung der Betriebstemperatur umfasst ein solcher Sensor ein Heizelement. Um die Messung von anderen Parametern, z.B. der Temperatur, nicht zu verfälschen, ist der Gassensor auf dem Teil der Leiterplatte, der aus dem Gehäuse herausragt, weit entfernt von den übrigen Sensoren angebracht. Eine mögliche Anordnung ist, dass die übrigen Sensoren weit in den Luftkanal hineinragen, während sich der Gassensor nahe am Rand des Luftkanals befindet.

Dazu kann der herausragende Teil der Leiterplatte stufenförmig ausgeprägt sein und zwei Stufen umfassen, wobei die zwei Stufen um die Längen l₁ und l₂ aus dem Gehäuse herausragen und l₁ > l₂ ist. Ein Abstand des Gassensors von der Öffnung im Gehäuse ist dann bevorzugt höchstens 25% von l₁, während ein Abstand der übrigen Sensoren von der Öffnung bevorzugt mindestens 75% von l₁ ist.

In einer bevorzugten Ausführung umfasst das Sensormodul eine Stromversorgung. Die Stromversorgung ist bevorzugt auf der Leiterplatte angebracht, bekommt den elektrischen Strom über den Stecker zugeführt und versorgt den Sensor bzw. die Sensoren mit Strom.

Darüber hinaus kann das Sensormodul eine Datenverarbeitung für Messwerte von dem mindestens einen Sensor umfassen. Die Datenverarbeitung kann beispielsweise eine Kalibration der Messwerte, eine Berechnung zusammengesetzter Daten von verschiedenen Sensoren und/oder eine Mittelung durchführen. Denkbar ist auch, dass bestimmte Grenzwerte implementiert sind, bei deren Über- oder Unterschreitung durch die Messwerte ein Warnsignal ausgegeben wird, beispielsweise bei Überschreitung eines Grenzwerts für die Konzentration eines gesundheitsschädlichen Gases in der Luft. Die Datenverarbeitung wird ebenfalls von der Stromversorgung mit Strom versorgt und die gemessenen und/oder verarbeiteten Daten werden über den Stecker bereitgestellt.

Außerdem kann das Sensormodul mindestens einen ESD-Bügel (electrostatic discharge) aus einem elektrisch leitenden Material auf der Leiterplatte umfassen, der den mindestens einen Sensor zumindest teilweise räumlich überbrückt. Der ESD-Bügel ist mit einem Massenanschluss der Leiterplatte verbunden und schützt den mindestens einen Sensor vor Beschädigung durch elektrostatische Entladung. Bei verschiedenen Ausführungsformen ist der ESD-Bügel an nur einer Stelle auf der Leiterplatte befestigt oder er ist z.B. auf zwei gegenüberliegenden Seiten des Sensors mit der Leiterplatte verbunden. In einer bevorzugten Ausführungsform befindet sich zwischen dem ESD-Bügel und dem Sensor ein Abstand, z.B. in der Größenordnung von 0.1 mm, der es ermöglicht, dass der Sensor direkt in Kontakt mit der umgebenden Luft steht. Im Speziellen ist ein ESD-Bügel angedacht, der sich flächenmäßig über den gesamten Sensor erstreckt, an der Stelle des sensitiven Elements aber ein Loch aufweist, um Luftkontakt für den Sensor zu ermöglichen. Bevorzugt ist der ESD-Bügel auf der Leiterplatte teilweise mit Füllmasse vergossen, was die Robustheit und die Lebensdauer des Sensormoduls erhöht.

Für die Füllmasse sind verschiedene Ausführungsformen denkbar. Erfindungsgemäss ist die Füllmasse ein Hot Melt, z.B. Henkel Technomelt PA 6771 oder Bostik Thermelt 181, oder ein UV-härtbarer Klebstoff. Diese haben den Vorteil, dass sie nicht unter hohem Druck angewendet werden müssen und damit eine Beschädigung des Sensors beim Vergießen vermieden wird. Es sind aber auch andere Klebstoffe, Gießmassen oder Harze denkbar. Beispielsweise kann ein gebräuchlicher Spritzguss verwendet werden, sofern es die Komponenten erlauben.

Ein weiterer Aspekt der vorliegenden Erfindung ist ein Verfahren zur Herstellung eines Sensormoduls zur Bestimmung einer Eigenschaft eines Fluids, beispielsweise zur Luftgütemessung, das die folgenden Schritte umfasst. (a) Es wird eine Leiterplatte bereitgestellt mit mindestens einem Sensor, der eine Messgröße des umgebenden Fluids, beispielsweise der umgebenden Luft misst; außerdem wird ein Gehäuse für die Leiterplatte bereitgestellt, das mindestens an einer Seite eine Öffnung für einen herausragenden Teil der Leiterplatte aufweist, (b) Die Leiterplatte wird in das Gehäuse eingeführt. (c) Eine Vorderseite, auf der sich der mindestens eine Sensor befindet, des herausragenden Teils der Leiterplatte wird mit einer Füllmasse vergossen, mit Ausnahme einer Aussparung für den mindestens einen Sensor. Ein solches Herstellungsverfahren hat den Vorteil, dass die einzelnen Teile modular bereitgestellt werden und leicht zusammengesetzt werden können. Durch das Vergießen entsteht ein abgedichtetes und mechanisch robustes Sensormodul.

In einer vorteilhaften Ausprägung des Verfahrens wird die Leiterplatte in Schritt b durch eine weitere Öffnung des Gehäuses eingeführt. Darüber hinaus kann das Einführen der Leiterplatte in Schritt b von einer Führungsvorrichtung und/oder einer Abschrägung im Gehäuse unterstützt werden. So ist ein problemloses Zusammensetzen des Sensormoduls gewährleistet und gleichzeitig sichergestellt, dass die Leiterplatte an einer vorgesehenen Position im Gehäuse sitzt.

Weiterhin umfasst Schritt b bevorzugt die folgenden Schritte: Die Leiterplatte wird durch die weitere Öffnung schräg in das Gehäuse eingeführt, wobei ein Abstand der Leiterplatte zur Abschrägung im Gehäuse grö-ßer gehalten wird als die Höhe des mindestens einen Sensors samt eines ESD-Bügels über der Leiterplatte; dieser Vorgang wird bevorzugt unterstützt durch die Führungsvorrichtung. Wenn der Teil der Leiterplatte mit dem mindestens einen Sensor durch die Öffnung aus dem Gehäuse herausragt, kann die Leiterplatte auf die Abschrägung gedrückt werden, bevorzugt unterstützt durch die Führungsvorrichtung. Die Leiterplatte kann über die Abschrägung in eine finale Position gekippt werden, wo sie bevorzugt von einer Arretierungsvorrichtung im Gehäuse arretiert wird.

In einer vorteilhaften Ausprägung wird in Schritt c zusätzlich eine der Vorderseite gegenüberliegende Rückseite des herausragenden Teils der Leiterplatte mit Ausnahme einer weiteren Aussparung, die dem mindestens einen Sensor gegenüberliegt, mit Füllmasse vergossen. Außerdem wird in Schritt c zusätzlich die Öffnung im Gehäuse mit Füllmasse vergossen. Vorteilhaft ist es auch, dass in Schritt c zusätzlich Hohlräume im Gehäuse und in der Leiterplatte samt dem mindestens einen Sensor vergossen werden, sodass lediglich eine Aussparung und eine weitere Aussparung für den mindestens einen Sensor sowie mögliche Hohlräume in dem mindestens einen Sensor bestehen bleiben. Dies erhöht die Robustheit des Sensormoduls weiter.

Bevorzugt wird das Gehäuse samt der eingeführten Leiterplatte in Schritt c für das Vergießen in eine Form transferiert, die einen Stempel für die Aussparung für den mindestens einen Sensor aufweist. Dabei kann der Stempel eine Kraft auf einen ESD-Bügel über dem mindestens einen Sensor ausüben und diesen vor dem Vergießen verformen. Vorteilhafterweise übt ein weiterer Stempel auf die Rückseite der Leiterplatte gegenüber dem ESD-Bügel eine Gegenkraft aus, sodass diese nicht verformt oder beschädigt wird.

Erfindungsgemäss ist die Füllmasse ein Hot Melt oder ein UV-härtbarer Klebstoff.

Weitere vorteilhafte Ausgestaltungen der Erfindung ergeben sich aus den abhängigen Ansprüchen, und den anhand der Zeichnungen nachfolgend dargestellten Ausführungsbeispielen. Es zeigen:
Fig. 1 eine Aufsicht von oben auf ein Sensormodul zur Bestimmung einer Eigenschaft eines Fluids, beispielsweise zur Luftgütemessung, mit einem Temperatur-/Feuchte- und einem Gassensor auf der Vorderseite einer Leiterplatte gemäß einer ersten Ausführungsform der Erfindung,
Fig. 2 eine Ansicht von der Seite auf das Sensormodul gemäß der ersten Ausführungsform,
Fig. 3 eine Ansicht von vorne auf das Sensormodul gemäß der ersten Ausführungsform,
Fig. 4 eine Ansicht von hinten auf das Sensormodul gemäß der ersten Ausführungsform,
Figs. 5 und 6 zwei Ansichten aus verschiedenen Blickwinkeln im Raum auf das Sensormodul gemäß der ersten Ausführungsform,
Fig. 7 eine Aufsicht von oben auf ein Sensormodul zur Bestimmung einer Eigenschaft eines Fluids, beispielsweise zur Luftgütemessung, mit einem Temperatur-/Feuchtesensor auf der Vorderseite einer Leiterplatte gemäß einer zweiten Ausführungsform der Erfindung,
Fig. 8 eine Aufsicht von oben auf ein Sensormodul zur Bestimmung einer Eigenschaft eines Fluids, beispielsweise zur Luftgütemessung, mit einem Temperatur-/Feuchtesensor auf der Vorderseite einer Leiterplatte gemäß einer dritten Ausführungsform der Erfindung,
Figs. 9 und 10 eine Seitenansicht auf einen vertikalen Schnitt durch das Gehäuse des Sensormoduls, während eine Leiterplatte mit einem Sensor bei der Herstellung gemäß einer Ausführungsform schräg eingeführt wird,
Fig. 11 einen vertikalen Schnitt durch das Gehäuse, während die Leiterplatte mit dem Sensor bei der Herstellung gemäß einer Ausführungsform auf eine Abschrägung gedrückt wird,
Fig. 12 einen vertikalen Schnitt durch das Gehäuse, während die Leiterplatte mit dem Sensor bei der Herstellung gemäß einer Ausführungsform über die Abschrägung in ihre finale Position gekippt wird,
Fig. 13 eine Aufsicht von unten auf ein horizontal durchgeschnittenes Sensormodul zur Bestimmung einer Eigenschaft eines Fluids, beispielsweise zur Luftgütemessung, mit einer Führungsvorrichtung und Ausrichtungspins für die Leiterplatte,
Figs. 14, 15 und 16 einen vertikalen Schnitt durch das Gehäuse mit verschiedenen Ausführungsformen einer Arretierungsvorrichtung für die Leiterplatte,
Figs. 17 und 18 einen vertikalen Schnitt durch das Gehäuse mit zwei Varianten einer Montage eines Steckers,
Fig. 19 eine Aufsicht von oben auf das Sensormodul mit Kennzeichnung eines vertikalen Schnitts A-B durch die Leiterplatte für die folgenden Fig.,
Figs. 20, 21 und 22 den vertikalen Schnitt AB durch die Leiterplatte.

### Wege zur Ausführung der Erfindung

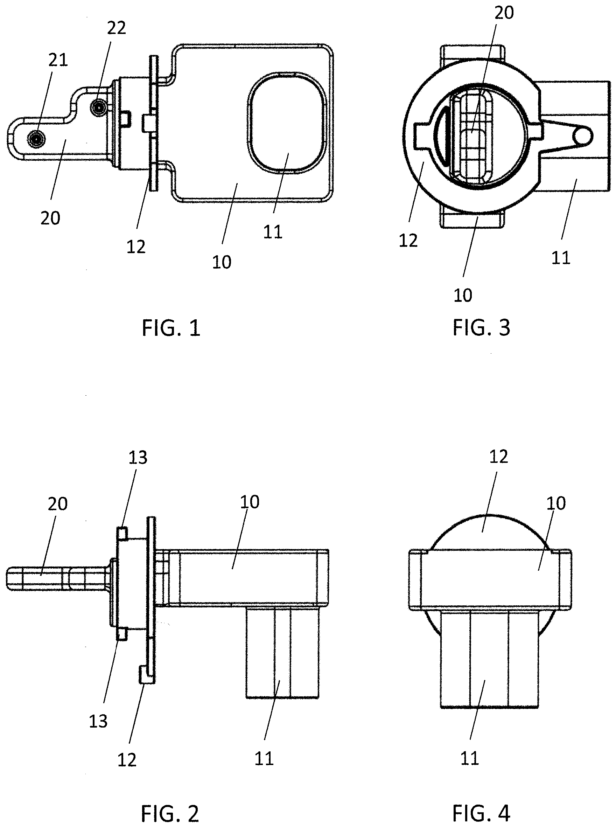

Fig. 1 zeigt eine Aufsicht von oben auf ein Sensormodul zur Bestimmung einer Eigenschaft eines Fluids, beispielsweise zur Luftgütemessung, mit einem Temperatur-/Feuchtesensor 21 und einem Gassensor 22 auf der Vorderseite einer Leiterplatte 20 gemäß einer ersten Ausführungsform der Erfindung. Der Sensor 21 ist ein integrierter Sensor für die Messung der Temperatur und der relativen Feuchtigkeit in der umgebenden Luft. Der Gassensor 22 ist ein MOX-Sensor (Metalloxid), der bevorzugt die Konzentration eines Gases, z.B. von NO2, SO2, O3, CO, VOC (volatile organic compounds), in der umgebenden Luft misst.

Die beiden Sensoren 21 und 22 sind auf einer Leiterplatte 20 angebracht und werden über diese mit Strom versorgt. Die Leiterplatte 20 ist zur Verstärkung und Dichtung mit einer Füllmasse eingekapselt, bevorzugt mit einem Hot Melt, z.B. Henkel Technomelt PA 6771 oder Bostik Thermelt 181. Die Einkapselung weist Aussparungen bei den Sensoren 21 und 22 auf, damit die umgebende Luft in Kontakt mit den sensitiven Elementen treten kann. Aus der Fig. 1 ist ersichtlich, dass die Sensoren 21 und 22 nicht mit Messkammern umgeben sind, sondern direkt dem umgebenden Fluid, beispielsweise der umgebenden Luft ausgesetzt sind, z.B. dem Luftstrom in einem Luftzufuhrkanal. Dies hat die Vorteile, dass die Messung nicht durch einen trägen Fluid-/ bzw. Luftaustausch in der Kammer verzögert wird, und dass die Messung nicht durch Ausgasungen, die sich in einer Kammer ansammeln könnten, verfälscht wird.

In der gezeigten Ausführungsform ist der Temperatur-/Feuchtesensor 21 nahe der Spitze der Leiterplatte positioniert, während sich der Gassensor 22 nahe des Gehäuses 10 befindet. Somit sind die beiden Sensoren 21 und 22 räumlich deutlich voneinander getrennt, was eine gegenseitige Beeinflussung verhindert, im Speziellen eine Verfälschung der Temperaturmessung durch ein Heizelement im Gassensor 22.

Auf der Leiterplatte 20 sind ebenfalls eine Stromversorgung und eine Datenverarbeitung für die Messwerte angebracht (beide nicht gezeigt, da innerhalb des Gehäuses 10 liegend). Diese befinden sich auf dem Teil der Leiterplatte 20, der nicht aus dem Gehäuse 10 herausragt. Das Gehäuse ist aus Kunststoff gefertigt, z.B. aus PP oder PBT, und schützt das Sensormodul vor mechanischer Beschädigung. Der Anschluss des Sensormoduls an eine externe Stromversorgung und Weiterverarbeitung der Daten ist über elektrische Kontakte in einem Stecker 11 bewerkstelligt. In der in Fig. 1 gezeigten Ausführungsform ist der Stecker 11 an einer als Oberseite bezeichneten Seite des Gehäuses 10 ausgebildet, während die Leiterplatte 20 an einer Seite, die als Vorderseite definiert ist, aus dem Gehäuse 10 herausragt. Abweichende Anordnungen sind möglich, z.B. dass sich der Stecker 11 an einer der Vorderseite gegenüberliegenden Hinterseite des Gehäuses 10 befindet.

Ein weiterer Teil des Gehäuses 10 in Fig. 1 ist der Bajonettverschluss 12 zur Befestigung des Sensormoduls an einem Träger, z.B. zur Anbringung an einem Luftzufuhrkanal für den Innenraum eines Kraftfahrzeugs oder eines Gebäudes. Durch die Befestigung mit einem Bajonettverschluss 12 kann eine luftdichte Verbindung mit gleichzeitiger Arretierung des Sensormoduls im Träger hergestellt werden. Andere Ausführungsformen des Befestigungsmittels sind aber ebenfalls möglich, z.B. über ein Gewinde.

Fig. 2 zeigt eine Ansicht von der Seite auf das Sensormodul gemäß der ersten Ausführungsform, während Fig. 3 eine Ansicht von vorne und Fig. 4 eine Ansicht von hinten illustrieren. In Fig. 2 wird deutlich, wie die Leiterplatte 20 mit den Sensoren an der Vorderseite aus dem Gehäuse 10 herausragt. Außerdem ist die Ausformung des Bajonettverschlusses 12 mit zwei Zapfen 13 zur Arretierung augenfällig. Die Ansicht von Fig. 4 von hinten bietet sich für einen Betrachter, der von außerhalb des Luftzufuhrkanals auf das montierte Sensormodul blickt.

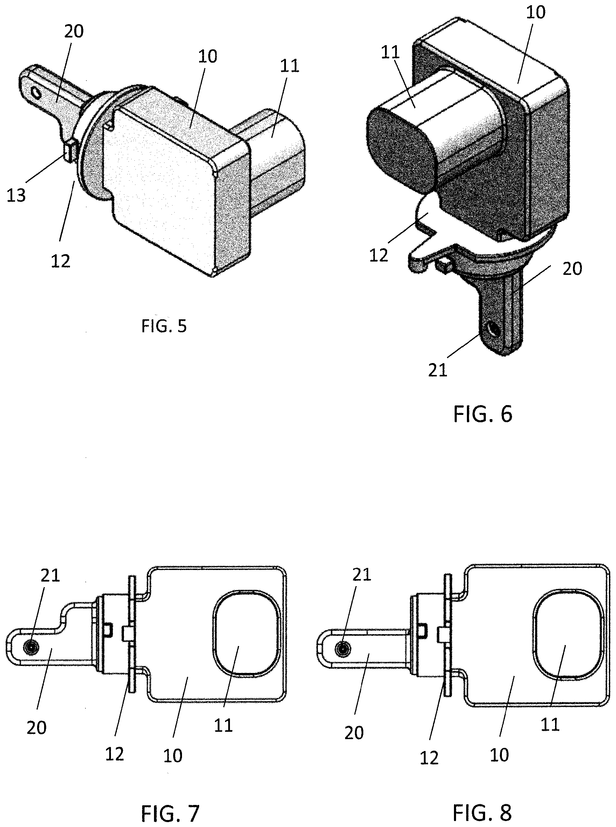

Fig. 5 zeigt eine räumliche Ansicht auf das Sensormodul mit Gehäuse 10 und eingegossener Leiterplatte 20 von schräg unten gemäß der ersten Ausführungsform von Fig. 1-4. Dabei ist der Zapfen 13 des Bajonettanschlusses 12 deutlich sichtbar.

Fig. 6 ergänzt dies durch eine räumliche Ansicht von schräg oben. In dieser Ansicht ist die Aussparung erkennbar, die sich in der Einkapselung um die Leiterplatte 20 über dem Sensor 21 befindet und direkten Kontakt eines sensitiven Elements des Sensors 21 mit dem umgebenden Fluid, beispielsweise der umgebenden Luft ermöglicht.

Fig. 7 zeigt eine Aufsicht von oben auf ein Sensormodul zur Bestimmung einer Eigenschaft eines Fluids, beispielsweise zur Luftgütemessung, mit einem Temperatur-/Feuchtesensor auf der Vorderseite einer Leiterplatte 20 gemäß einer zweiten Ausführungsform der Erfindung. Die zweite Ausführungsform umfasst zum großen Teil die gleichen Komponenten wie die erste Ausführungsform in Fig. 1-6. Dies sind ein Gehäuse 10 mit Ausformungen für einen Stecker 11 und einen Bajonettverschluss 12, sowie eine eingekapselte Leiterplatte 20. Im Unterschied zu Fig. 1-6 zeigt Fig. 7 nur einen Sensor 21. In einer Ausführungsform ist dies wiederum ein integrierter Temperatur-/Feuchtesensor.

Fig. 8 illustriert eine Aufsicht von oben auf ein Sensormodul zur Bestimmung einer Eigenschaft eines Fluids, beispielsweise zur Luftgütemessung, mit einem Temperatur-/Feuchtesensor auf der Vorderseite einer Leiterplatte 20 gemäß einer dritten Ausführungsform der Erfindung. Dabei besteht der Unterschied zu den ersten beiden Ausführungsformen in Fig. 1-6 bzw. 7 in der Form der eingekapselten Leiterplatte 20. Der Teil der Leiterplatte 20, der aus dem Gehäuse heraus- und damit im montierten Zustand in den Luftzufuhrkanal hineinragt, ist in Fig. 8 weniger breit. Dies führt zu einer geringeren Beeinflussung des Luftstroms im Kanal, bzw. des zu messenden Fluids. Wie in der zweiten Ausführungsform ist auf der Leiterplatte 20 bevorzugt auch hier ein integrierter Temperatur-/Feuchtesensor angebracht. Denkbar ist aber für alle Ausführungsformen auch die Verwendung nur eines einzelnen Temperatursensors, wenn die Werte der relativen Feuchtigkeit nicht von Interesse sind.

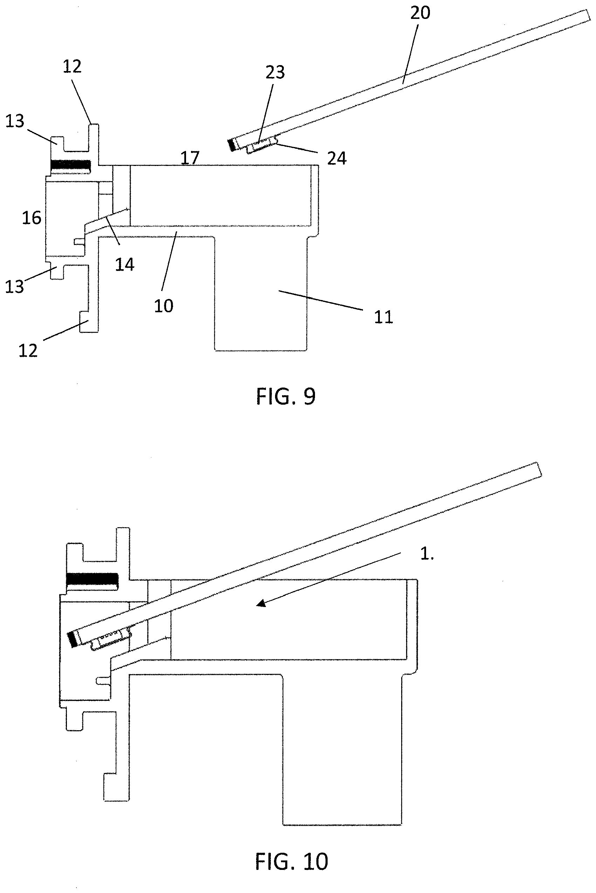

Figs. 9-12 zeigen eine Seitenansicht eines Gehäuses 10 des Sensormoduls bei der Herstellung, während eine Leiterplatte 20 mit einem Sensor 23 vor dem Vergie-ßen gemäß einer Ausführungsform eingeführt wird. Das Gehäuse 10 mit Ausformungen für Stecker 11 und Bajonettverschluss 12 samt Zapfen 13 entspricht dabei weitgehend dem in Fig. 2 gezeigten. Zusätzlich ist in Fig. 9-12 eine Abschrägung 14 erkennbar, deren Zweck im Folgenden erläutert wird.

Die Leiterplatte 20 ist in Fig. 9-12 noch nicht vergossen. Daher ist der Sensor 23 als aufgesetzter Chip sichtbar. Außerdem wird der Sensor 23 von einem ESD-Bügel 24 (electrostatic discharge) überspannt, welcher ihn vor Beschädigung durch elektrostatische Entladung schützt. Auch der ESD-Bügel 23 ist nach dem Vergießen zum großen Teil eingekapselt, was die mechanische Stabilität des Sensormoduls erhöht.

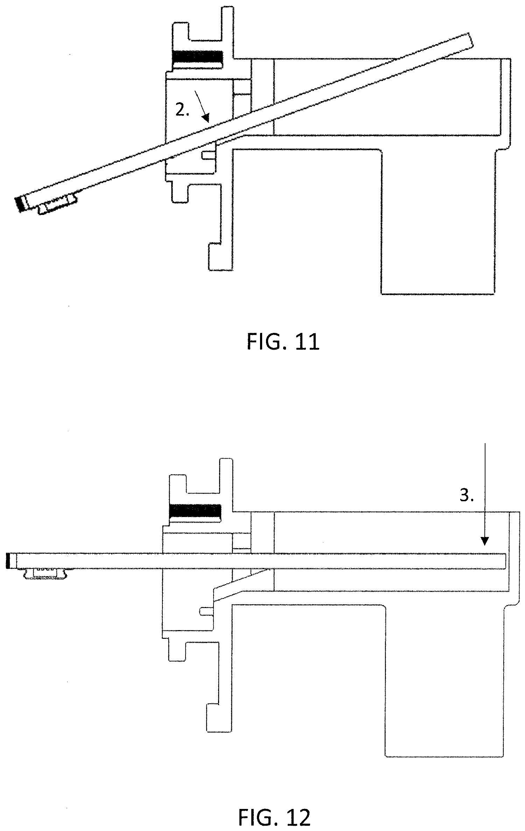

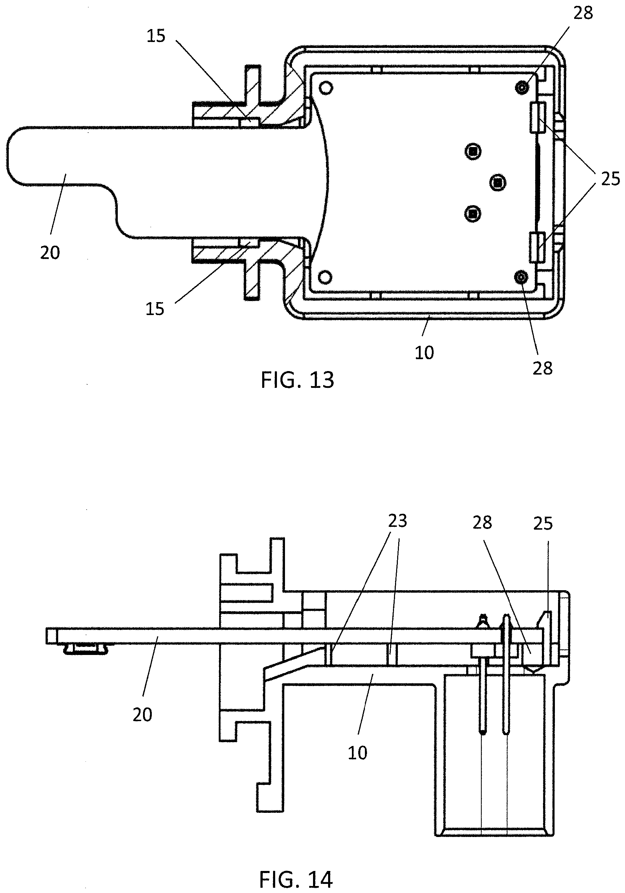

Da der Sensor 23 und der ESD-Bügel 24 vor dem Vergießen leicht beschädigt werden können, wird die Leiterplatte 20 im ersten Montageschritt in Fig. 9 schräg und mit genügend Abstand zur Abschrägung 14 eingeführt, sodass der Sensor 23 und der ESD-Bügel 24 die Abschrägung 14 nicht berühren. In Fig. 10 wird gezeigt, wie die Leiterplatte 20 aus der Öffnung 16 in der Vorderseite des Gehäuses 10 herausgeschoben wird, sodass der Teil der Leiterplatte 20 mit dem Sensor 23 im Fig. 11 aus dem Gehäuse 10 herausragt. Dabei wird die Leiterplatte 20 von einer Führungsvorrichtung 15 geführt, die in Fig. 13 gezeigt ist. In Fig. 11 wird die Leiterplatte dann gegen die Abschrägung 14 gedrückt. Das Einführen der Leiterplatte 20 wird in Fig. 12 abgeschlossen, indem sie über die Abschrägung 14 gekippt wird und damit mit Ausnahme des vorne herausragenden Teils mit dem Sensor 23 vollständig im Gehäuse 10 zu liegen kommt.

Zum Abschluss wird die in Fig. 12 gezeigte Anordnung vergossen. Dazu wird die gesamte Anordnung in senkrechter Ausrichtung wie beispielsweise in Fig. 1 gezeigt in eine Form gesetzt, die für jeden Sensor einen Stempel umfasst, der die Aussparung für den Sensor frei hält. Nun werden sowohl die weitere Öffnung 17 des Gehäuses 10, durch welche die Leiterplatte 20 in Fig. 9-11 eingeführt wurde, verfüllt als auch der aus der Öffnung 16 herausragende Teil der Leiterplatte 20 mit dem Sensor 23 und dem ESD-Bügel 24 mit Ausnahme der Aussparung eingekapselt.

Als Füllmasse können verschiedene Materialien verwendet werden, bevorzugt ist aber ein Hot Melt. Der Vorteil eines Hot Melts ist, dass beim Verkleben geringere Drücke angewendet werden müssen als bei anderen Methoden wie beispielsweise dem Vergießen mit Spritzguss. Als Füllmasse sind aber beispielsweise auch UV-härtbare Harze denkbar. Am Ende des Fertigungsprozesses steht ein kompaktes und robustes Sensormodul.

Fig. 13 zeigt eine Aufsicht von unten auf ein horizontal durchgeschnittenes Sensormodul zur Bestimmung einer Eigenschaft eines Fluids, beispielsweise zur Luftgütemessung. Die Leiterplatte 20 wird durch die Führungsvorrichtung 15 gegen seitliches Verschieben im Gehäuse 10 gesichert. Außerdem unterstützt die Führungsvorrichtung 15 den oben beschriebenen Einführungsvorgang. Weiterhin helfen zwei Ausrichtungspins 28 beim korrekten Ausrichten der Leiterplatte 20 und Klammern 25 dienen als Arretierungsvorrichtung der Leiterplatte 20 in der gewünschten Position.

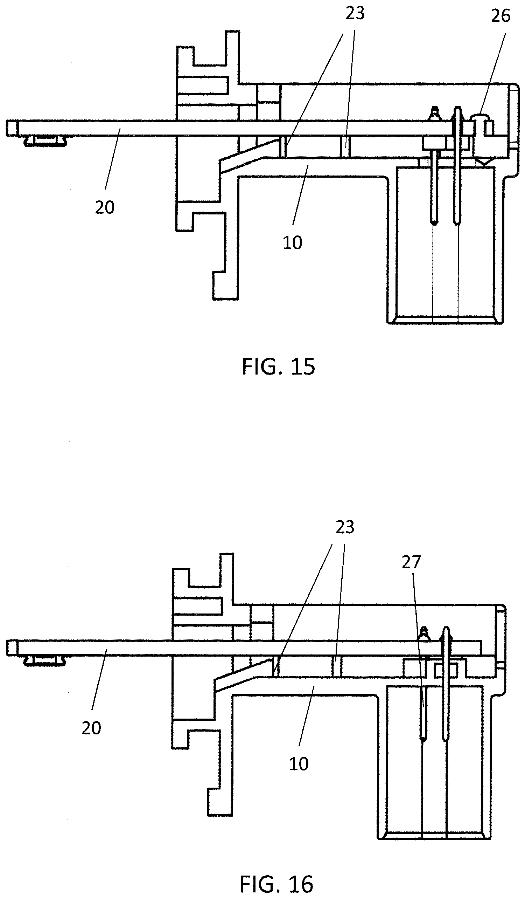

Figs. 14, 15 und 16 illustrieren einen vertikalen Schnitt durch das Gehäuse 10 mit verschiedenen Ausführungsformen der Arretierungsvorrichtung für die Leiterplatte 20. Nach oben hin wird die Leiterplatte 20 in vertikaler Richtung dabei von Stützen 23 abgestützt. Nach unten hin dienen in Fig. 14 die Klammern 25 zum Festhalten der Leiterplatte 20. In Fig. 15 dient zur Arretierung nach unten hin ein Pin 26, dessen Ende verschweißt wird, um die Leiterplatte 20 zu halten. In Fig. 16 wird die Arretierung nach unten hin dadurch erreicht, dass ein fest im Gehäuse 10 angebrachter Pin 27 des Steckers 11 mit der Leiterplatte 20 verlötet oder verpresst wird.

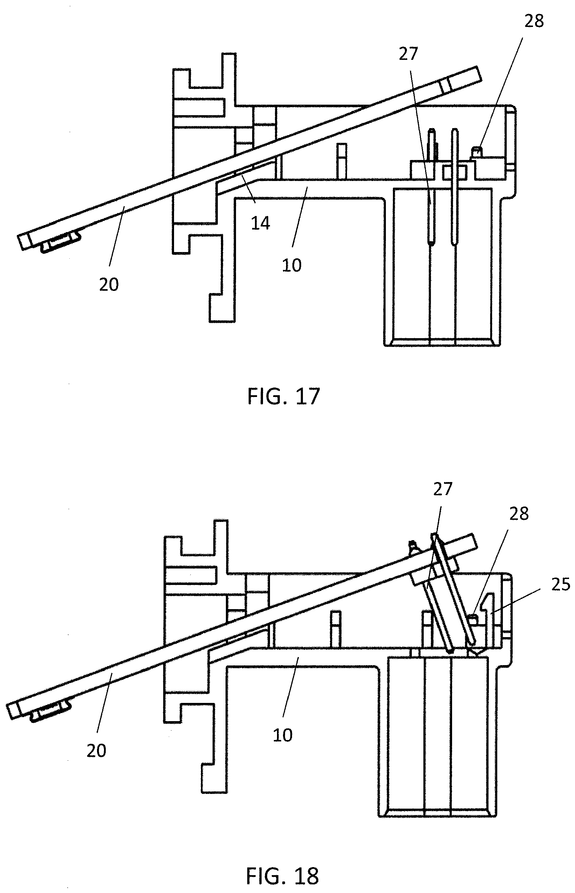

Figs. 17 und 18 zeigen einen vertikalen Schnitt durch das Gehäuse mit zwei Varianten zur Anbringung von Pins 27 eines Steckers 11. In Fig. 17 sind die Pins 27 bereits vor dem Einführen der Leiterplatte 20 in das Gehäuse 10 fest im Gehäuse 10 montiert. Beim Kippen der Leiterplatte über die Abschrägung 14 durchstoßen die Pins 27 die Leiterplatte 20 dann in dafür vorgesehenen Löchern in der Leiterplatte 20. Abschließend werden die Pins 27 mit der Leiterplatte 20 verlötet oder bereits durch das Einpressen fixiert.

In Fig. 18 sind die Pins 27 vor dem Einführen der Leiterplatte 20 in das Gehäuse 10 bereits auf der Leiterplatte 20 verlötet oder in die Leiterplatte 20 eingepresst. Beim Kippen der Leiterplatte 20 über die Abschrägung 14 werden sie dann bis zu ihrer gewünschten Lage in den Stecker 11 eingeführt.

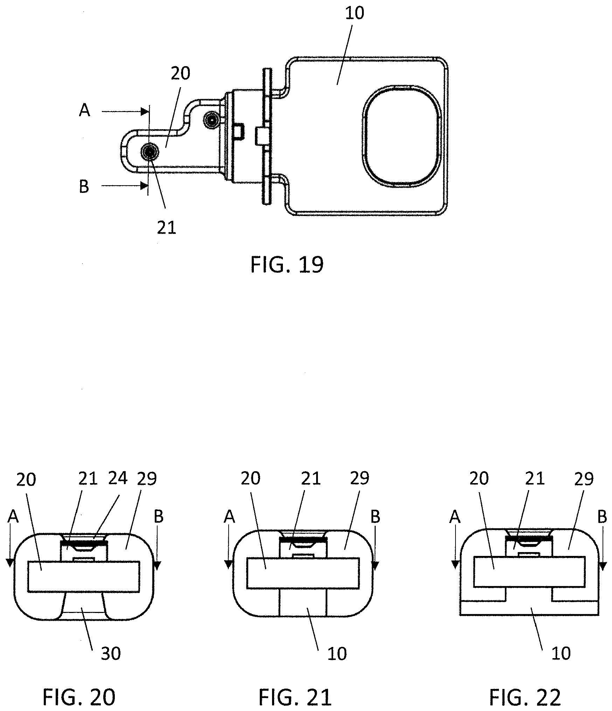

Fig. 19 zeigt eine Aufsicht von oben auf das Sensormodul mit Gehäuse 10 und Leiterplatte 20 sowie Kennzeichnung eines vertikalen Schnitts A-B durch die Leiterplatte 20. In Figs. 20, 21 und 22 sind verschiedene Ausführungsformen des Vergusses der Leiterplatte 20 mit der Füllmasse 29 durch vertikale Schnitte A-B durch die Leiterplatte 20 dargestellt. Bei der Ausführungsform von Fig. 20 ist die komplette Leiterplatte 20 mit Ausnahme einer Aussparung für den Sensor 21 auf der Vorderseite und einer weiteren Aussparung 30 auf der Rückseite mit der Füllmasse 29 eingekapselt. Die weitere Aussparung liegt dem Sensor 21 gegenüber und kommt von einem weiteren Stempel, der beim Vergießen der Leiterplatte 20 mit der Füllmasse 29 einen Gegendruck auf die Rückseite der Leiterplatte 20 ausübt, während auf der Vorderseite ein Stempel Druck auf den ESD-Bügel 24 ausübt.

In den Ausführungsformen von Figs. 21 und 22 befindet sich dem Sensor 21 gegenüber auf der Rückseite der Leiterplatte 20 keine weitere Aussparung, sondern ein Teil des Gehäuses 10 ist entlang der Leiterplatte 20 bis dorthin durchgezogen. Dieser Gehäuseteil unterstützt den herausragenden Teil der Leiterplatte 20 und macht das Sensormodul so robuster. In Fig. 21 beschränkt sich die seitliche Ausdehnung dieses Gehäuseteils entlang des Schnitts A-B auf die Breite des gegenüberliegenden Sensors 21. In Fig. 22 weist der Gehäuseteil im Schnitt A-B die Form eines auf dem Kopf stehenden "T" auf, dessen Breite die Breite der Leiterplatte 20 übertrifft. Der Hohlraum zwischen dem Querstrich des "T" und der Leiterplatte 20 wird beim Einkapseln mit Füllmasse 29 vergossen. Diese Anordnung macht den herausragenden Teil der Leiterplatte 20 noch unempfindlicher gegen mechanische Einwirkungen.

## Patentansprüche

1. Sensormodul zur Bestimmung einer Eigenschaft eines Fluids, insbesondere zur Luftgütemessung, umfassend
- eine Leiterplatte (20),
- mindestens einen Sensor (21, 22) auf der Leiterplatte (20) zum Aufnehmen einer Messgröße des Fluids, insbesondere der umgebenden Luft, und
- ein Gehäuse (10) für die Leiterplatte (20),
wobei ein Teil der Leiterplatte (20) aus einer Öffnung im Gehäuse (10) herausragt,
wobei sich der mindestens eine Sensor (21, 22) auf einer Vorderseite des herausragenden Teils der Leiterplatte (20) befindet,
wobei zumindest die Vorderseite des herausragenden Teils der Leiterplatte (20) mit Ausnahme einer Aussparung für den mindestens einen Sensor (21, 22) mit einer Füllmasse (29) vergossen ist,
**dadurch gekennzeichnet, dass** zusätzlich zumindest die Öffnung (16) im Gehäuse (10) mit Füllmasse (29) vergossen ist,
wobei die Füllmasse (29) ein Hot Melt oder ein UV-härtbarer Klebstoff ist.

2. Sensormodul nach einem der vorangehenden Ansprüche, das Gehäuse (10) umfassend ein Befestigungsmittel (12) zur Anbringung an einem Luftkanal oder in einem Wasserkasten,
insbesondere wobei sich das Befestigungsmittel (12) an der Seite des Gehäuses (10) mit der Öffnung (16) befindet, und/oder
insbesondere wobei das Befestigungsmittel (12) einen Bajonettverschluss umfasst.

3. Sensormodul nach einem der vorangehenden Ansprüche, das Gehäuse (10) umfassend eine weitere Öffnung (17) auf einer Seite, die an die Seite mit der Öffnung (16) angrenzt oder dieser gegenüber liegt,
insbesondere wobei die weitere Öffnung (17) mit Füllmasse (29) vergossen ist.

4. Sensormodul nach Anspruch 3, das Gehäuse (10) umfassend eine Führungsvorrichtung (15) zum Einführen der Leiterplatte (20) durch die weitere Öffnung (17) in das Gehäuse (10),
insbesondere wobei die Führungsvorrichtung (15) eine Abschrägung (14) im Gehäuse (10) umfasst, die schräg zur Seite mit der weiteren Öffnung (17) angeordnet ist.

5. Sensormodul nach einem der vorangehenden Ansprüche, wobei der mindestens eine Sensor (21, 22) einen Temperatursensor enthält, und/oder
wobei der mindestens eine Sensor (21, 22) einen Feuchtesensor enthält,
insbesondere wobei der Temperatursensor und der Feuchtesensor in einem einzigen Chip integriert sind.

6. Sensormodul nach einem der vorangehenden Ansprüche, wobei der mindestens eine Sensor (21, 22) einen Gassensor enthält,
insbesondere wobei der Gassensor eine Konzentration eines Gases in der umgebenden Luft misst, und/oder
insbesondere wobei der Gassensor ein MOX-Sensor ist.

7. Sensormodul nach einem der vorangehenden Ansprüche, wobei der herausragende Teil der Leiterplatte (20) stufenförmig ausgeprägt ist und zwei Stufen umfasst,
wobei die zwei Stufen um die Längen l₁ und l₂ aus dem Gehäuse (10) herausragen,
wobei l₁ > l₂ ist.

8. Sensormodul nach den Ansprüchen 5 bis 7, wobei ein Abstand des Gassensors von der Öffnung (16) im Gehäuse (10) höchstens 25% von l₁ beträgt, und
wobei ein Abstand des Temperatursensors und/oder des Feuchtesensors von der Öffnung (16) im Gehäuse mindestens 75% von l₁ beträgt.

9. Sensormodul nach einem der vorangehenden Ansprüche, umfassend eine Datenverarbeitung für Messwerte von dem mindestens einen Sensor (21, 22) auf der Leiterplatte (20).

10. Sensormodul nach einem der vorangehenden Ansprüche, umfassend mindestens einen ESD-Bügel (24) aus einem elektrisch leitenden Material auf der Leiterplatte (20), der den mindestens einen Sensor (21, 22) zumindest teilweise räumlich überbrückt und mit einem Massenanschluss der Leiterplatte (20) verbunden ist, zum Schutz des Sensors (21, 22) vor Beschädigung durch elektrostatische Entladung,
insbesondere wobei der ESD-Bügel (24) teilweise mit Füllmasse (29) vergossen ist.

11. Ein Verfahren zur Herstellung eines Sensormoduls zur Bestimmung einer Eigenschaft eines Fluids, beispielsweise zur Luftgütemessung, umfassend die folgenden Schritte:
a. Bereitstellen einer Leiterplatte (20) mit mindestens einem Sensor (23), der eine Messgröße des umgebenden Fluids, beispielsweise der umgebenden Luft misst, und eines Gehäuses (10) für die Leiterplatte (20), das mindestens an einer Seite eine Öffnung (16) für einen herausragenden Teil der Leiterplatte (20) aufweist,
b. Einführen der Leiterplatte (20) in das Gehäuse (10),
c. Vergießen einer Vorderseite, auf der sich der mindestens eine Sensor (23) befindet, des herausragenden Teils der Leiterplatte (20) mit Ausnahme einer Aussparung für den mindestens einen Sensor (23) mit einer Füllmasse (29), wobei zusätzlich zumindest die Öffnung (16) im Gehäuse (10) mit Füllmasse (29) vergossen wird, wobei die Füllmasse (29) ein Hot Melt oder ein UV-härtbarer Klebstoff ist.

12. Das Verfahren nach Anspruch 11, wobei die Leiterplatte (20) in Schritt b durch eine weitere Öffnung (17) des Gehäuses (10) eingeführt wird, und/oder
wobei das Einführen der Leiterplatte (20) in Schritt b von einer Führungsvorrichtung (15) und/oder einer Abschrägung (14) im Gehäuse (10) unterstützt wird.

13. Das Verfahren nach einem der Ansprüche 11 oder 12, wobei Schritt b die folgenden Schritte umfasst:
- Schräges Einführen der Leiterplatte (20) in das Gehäuse (10) durch die weitere Öffnung (17), wobei ein Abstand der Leiterplatte (20) zur Abschrägung (14) im Gehäuse (10) größer gehalten wird als die Höhe des mindestens einen Sensors (23) samt eines ESD-Bügels (24) über der Leiterplatte (20), bevorzugt unterstützt durch die Führungsvorrichtung (15),
- wenn der Teil der Leiterplatte (20) mit dem mindestens einen Sensor (23) durch die Öffnung (16) aus dem Gehäuse (10) herausragt, Drücken der Leiterplatte (20) auf die Abschrägung (14), bevorzugt unterstützt durch die Führungsvorrichtung (15),
- Kippen der Leiterplatte (20) über die Abschrägung (14) in eine finale Position, wo sie bevorzugt von einer Arretierungsvorrichtung (25, 26, 27) im Gehäuse (10) arretiert wird.

14. Das Verfahren nach einem der Ansprüche 11 bis 13, wobei das Gehäuse (10) samt der eingeführten Leiterplatte (20) in Schritt c für das Vergießen in eine Form transferiert wird, die einen Stempel für die Aussparung für den mindestens einen Sensor (23) aufweist,
insbesondere wobei der Stempel eine Kraft auf einen ESD-Bügel (24) über dem mindestens einen Sensor (23) ausübt und diesen vor dem Vergießen verformt, und/oder
insbesondere wobei ein weiterer Stempel auf die Rückseite der Leiterplatte (20) gegenüber dem ESD-Bügel (24) eine Gegenkraft ausübt.

## Claims

1. Sensor module for determining a property of a fluid, in particular for measuring air quality, comprising
- a printed circuit board (20),
- at least one sensor (21, 22) on the printed circuit board (20) for recording a measured value of the fluid, in particular of the ambient air, and
- a housing (10) for the printed circuit board (20),
wherein a part of the printed circuit board (20) protrudes from an opening in the housing (10),
wherein the at least one sensor (21, 22) is located on a front side of the protruding part of the printed circuit board (20),
wherein at least the front side of the protruding part of the printed circuit board (20), with the exception of a recess for the at least one sensor (21, 22), is moulded with a filling compound (29),
**characterised in that**
in addition, at least the opening (16) in the housing (10) is moulded with filling compound (29),
wherein the filling compound (29) is a hot melt or a UV-curable adhesive.

2. Sensor module according to one of the preceding claims, the housing (10) comprising a fastening means (12) for attachment to an air duct or in a water tank,
in particular wherein the fastening means (12) is located on the side of the housing (10) with the opening (16), and/or
in particular wherein the fastening means (12) comprises a bayonet catch.

3. Sensor module according to one of the preceding claims, the housing (10) comprising a further opening (17) on a side which is adjacent to or opposite the side with the opening (16),
in particular wherein the further opening (17) is moulded with filling compound (29).

4. Sensor module according to claim 3, the housing (10) comprising a guide device (15) for inserting the printed circuit board (20) through the further opening (17) in the housing (10),
in particular wherein the guide device (15) comprises a bevelled edge (14) in the housing (10), which is arranged at an angle to the side with the further opening (17).

5. Sensor module according to one of the preceding claims, wherein the at least one sensor (21, 22) comprises a temperature sensor, and/or
wherein the at least one sensor (21, 22) comprises a humidity sensor,
in particular wherein the temperature sensor and the humidity sensor are integrated in a single chip.

6. Sensor module according to one of the preceding claims, wherein the at least one sensor (21, 22) comprises a gas sensor,
in particular wherein the gas sensor measures a concentration of a gas in the ambient air, and/or
in particular wherein the gas sensor is a MOX sensor.

7. Sensor module according to one of the preceding claims, wherein the protruding part of the printed circuit board (20) is step-shaped and comprises two steps,
wherein the two steps protrude from the housing (10) by the lengths l₁ and l₂ ,
wherein l₁ > l₂.

8. Sensor module according to claims 5 to 7, wherein a distance of the gas sensor from the opening (16) in the housing (10) is at most 25% of l₁ , and
wherein a distance of the temperature sensor and/or the humidity sensor from the opening (16) in the housing is at least 75% of l₁.

9. Sensor module according to one of the preceding claims, comprising data processing for measured values from the at least one sensor (21, 22) on the printed circuit board (20).

10. Sensor module according to one of the preceding claims, comprising at least one ESD bracket (24) made of an electrically conductive material on the printed circuit board (20), which at least partially spatially bridges the at least one sensor (21, 22) and is connected to a ground connection of the printed circuit board (20), for protecting the sensor (21, 22) from damage by electrostatic discharge,
in particular wherein the ESD bracket (24) is partially moulded with filling compound (29).

11. A method of manufacturing a sensor module for determining a property of a fluid, for example for air quality measurement, comprising the following steps:
a. Providing a printed circuit board (20) with at least one sensor (23), which measures a measured value of the surrounding fluid, for example of the surrounding air, and a housing (10) for the printed circuit board (20), which has an opening (16) for a protruding part of the printed circuit board (20) on at least one side,
b. Inserting the printed circuit board (20) into the housing (10),
c. Moulding of a front side, on which the at least one sensor (23) is located, of the protruding part of the printed circuit board (20), with the exception of a recess for the at least one sensor (23), with a filling compound (29), at least the opening (16) in the housing (10) being additionally moulded with filling compound (29), the filling compound (29) being a hot melt or a UV-curable adhesive.

12. The method according to claim 11, wherein the printed circuit board (20) is inserted in step b through a further opening (17) of the housing (10), and/or
wherein the insertion of the printed circuit board (20) in step b is supported by a guide device (15) and/or a bevelled edge (14) in the housing (10).

13. The method according to one of claims 11 or 12, wherein step b comprises the following steps:
- Inclined insertion of the printed circuit board (20) into the housing (10) through the further opening (17), wherein a distance between the printed circuit board (20) and the bevelled edge (14) in the housing (10) is kept greater than the height of the at least one sensor (23) together with an ESD bracket (24) over the printed circuit board (20), preferably supported by the guide device (15),
- When the part of the printed circuit board (20) with the at least one sensor (23) protrudes from the housing (10) through the opening (16), pressing the printed circuit board (20) onto the bevelled edge (14), preferably supported by the guide device (15),
- Tilting the printed circuit board (20) over the bevelled edge (14) into a final position, where it is preferably locked in the housing (10) by a locking device (25, 26, 27).

14. The method according to one of claims 11 to 13, wherein the housing (10) together with the inserted printed circuit board (20) is transferred in step c for moulding into a mould which has a punch for the recess for the at least one sensor (23),
in particular wherein the punch exerts a force on an ESD bracket (24) over the at least one sensor (23) and deforms it before moulding, and/or
in particular wherein a further punch exerts a counterforce on the rear side of the printed circuit board (20) with respect to the ESD bracket (24).

## Revendications

1. Module de capteur pour la détermination d'une propriété d'un fluide, en particulier pour la mesure de la qualité de l'air, comprenant
- une carte de circuit imprimé (20),
- au moins un capteur (21, 22) sur la carte de circuit imprimé (20) pour enregistrer une valeur de mesure du fluide, en particulier de l'air ambiant, et
- un boîtier (10) pour la carte de circuit imprimé (20),
une partie de la carte de circuit imprimé (20) faisant saille d'une ouverture dans le boîtier (10),
le au moins un capteur (21, 22) se trouvant sur une face avant de la partie en saillie de la carte de circuit imprimé (20),
dans lequel au moins la face avant de la partie en saillie de la carte de circuit imprimé (20), à l'exception d'un évidement pour le au moins un capteur (21, 22), est moulée avec une masse de remplissage (29),
**caractérisé en ce qu'**en outre, au moins l'ouverture (16) dans le boîtier (10) est scellée avec une masse de remplissage (29),
la masse de remplissage (29) étant une colle thermofusible ou une colle durcissable aux UV.

2. Module de capteur selon l'une des revendications précédentes, le boîtier (10) comprenant un moyen de fixation (12) destiné à être monté sur un conduit d'air ou dans une boîte à eau,
en particulier, le moyen de fixation (12) étant situé sur le côté du boîtier (10) comportant l'ouverture (16), et/ou
en particulier dans lequel le moyen de fixation (12) comprend une fermeture à baïonnette.

3. Module de détection selon l'une des revendications précédentes, le boîtier (10) comprenant une ouverture supplémentaire (17) sur un côté adjacent ou opposé au côté comportant l'ouverture (16),
en particulier dans lequel l'ouverture supplémentaire (17) est scellée avec une masse de remplissage (29).

4. Module de capteur selon la revendication 3, le boîtier (10) comprenant un dispositif de guidage (15) pour l'introduction de la carte de circuit imprimé (20) dans le boîtier (10) par l'ouverture supplémentaire (17),
en particulier, le dispositif de guidage (15) comprenant un chanfrein (14) dans le boîtier (10) qui est disposé de manière oblique sur le côté avec l'ouverture supplémentaire (17).

5. Module de capteur selon l'une des revendications précédentes, dans lequel le au moins un capteur (21, 22) comprend un capteur de température, et/ou
l'au moins un capteur (21, 22) comprend un capteur d'humidité,
en particulier dans lequel le capteur de température et le capteur d'humidité sont intégrés dans une seule puce.

6. Module de capteur selon l'une des revendications précédentes, dans lequel le au moins un capteur (21, 22) contient un capteur de gaz,
en particulier dans lequel le capteur de gaz mesure une concentration d'un gaz dans l'air ambiant, et/ou
en particulier dans lequel le capteur de gaz est un capteur de MOX.

7. Module de capteur selon l'une des revendications précédentes, dans lequel la partie saillante de la carte de circuit imprimé (20) est étagée et comprend deux étages,
les deux étages faisant saillie du boîtier (10) par des longueurs l₁ et l₂,
où l₁ > l₂.

8. Module de capteur selon les revendications 5 à 7, dans lequel une distance du capteur de gaz par rapport à l'ouverture (16) dans le boîtier (10) est au plus égale à 25% de l₁, et
Dans lequel une distance du capteur de température et/ou du capteur d'humidité par rapport à l'ouverture (16) dans le boîtier est d'au moins 75% de l₁.

9. Module de capteur selon l'une des revendications précédentes, comprenant un traitement de données pour les valeurs de mesure de le au moins un capteur (21, 22) sur la carte de circuit imprimé (20).

10. Module de détection selon l'une des revendications précédentes, comprenant au moins un étrier ESD (24) en un matériau électriquement conducteur sur la carte de circuit imprimé (20), qui recouvre au moins partiellement dans l'espace le au moins un capteur (21, 22) et qui est relié à une borne de masse de la carte de circuit imprimé (20), pour protéger le capteur (21, 22) contre un endommagement par décharge électrostatique,
en particulier l'étrier ESD (24) étant partiellement coulé avec une masse de remplissage (29).

11. Un procédé de fabrication d'un module de capteur pour déterminer une propriété d'un fluide, par exemple pour mesurer la qualité de l'air, comprenant les étapes suivantes :
a. Préparer une carte de circuit imprimé (20) avec au moins un capteur (23) qui mesure une valeur de mesure du fluide ambiant, par exemple de l'air ambiant, et un boîtier (10) pour la carte de circuit imprimé (20), qui présente au moins sur un côté une ouverture (16) pour une partie saillante de la carte de circuit imprimé (20),
b. Insérer la carte de circuit imprimé (20) dans le boîtier (10),
c. Couler une face avant, sur laquelle se trouve le au moins un capteur (23), de la partie saillante de la carte à circuit imprimé (20), à l'exception d'un évidement pour le au moins un capteur (23), avec une masse de remplissage (29), au moins l'ouverture (16) dans le boîtier (10) étant en outre coulée avec une masse de remplissage (29), la masse de remplissage (29) étant une colle hot melt ou une colle durcissable aux UV.

12. Le procédé selon la revendication 11, dans lequel la carte de circuit imprimé (20) est insérée à l'étape b à travers une autre ouverture (17) du boîtier (10), et/ou
dans lequel l'introduction de la carte de circuit imprimé (20) à l'étape b est assistée par un dispositif de guidage (15) et/ou un chanfrein (14) dans le boîtier (10).

13. Le procédé selon l'une des revendications 11 ou 12, dans lequel l'étape b comprend les étapes suivantes :
- Introduire de manière oblique la carte de circuit imprimé (20) dans le boîtier (10) à travers l'autre ouverture (17), une distance de la carte de circuit imprimé (20) par rapport au chanfrein (14) dans le boîtier (10) étant maintenue supérieure à la hauteur d'au moins un capteur (23) y compris un étrier ESD (24) au-dessus de la carte de circuit imprimé (20), de préférence assistée par le dispositif de guidage (15),
- lorsque la partie de la carte de circuit imprimé (20) comportant au moins un capteur (23) fait saillie du boîtier (10) par l'ouverture (16), presser la carte de circuit imprimé (20) sur le chanfrein (14), de préférence avec l'aide du dispositif de guidage (15),
- Basculer la carte de circuit imprimé (20) sur le chanfrein (14) dans une position finale, où elle est bloquée de préférence par un dispositif de blocage (25, 26, 27) dans le boîtier (10).

14. Le procédé selon l'une des revendications 11 à 13, dans lequel le boîtier (10) avec la carte de circuit imprimé (20) insérée est transféré à l'étape c pour le coulage dans un moule qui présente un poinçon pour l'évidement pour le au moins un capteur (23),
en particulier le poinçon exerçant une force sur un étrier ESD (24) au-dessus du au moins un capteur (23) et le déformant avant le coulage, et/ou
en particulier un autre poinçon exerçant une force antagoniste sur la face arrière de la carte de circuit imprimé (20) par rapport à l'étrier ESD (24).
